# EUROPÄISCHE PATENTSCHRIFT

(11) **EP 1 149 088 B1**
(45) Veröffentlichungstag und Bekanntmachung des Hinweises auf die Patenterteilung: **23.04.2003**
(21) Anmeldenummer: 00910634.5
(22) Anmeldetag: 01.02.2000
(51) Int. Cl.: C07D 327/10, C07C 301/00

(54) **VERFAHREN ZUR HERSTELLUNG VON DIMETHYLSULFIT**
METHOD OF PRODUCING DIMETHYL SULFITE
PROCEDE PERMETTANT DE PRODUIRE DU DIMETHYL-SULFURE

(30) Priorität: 03.02.1999 DE 19904261
(43) Veröffentlichungstag der Anmeldung: 31.10.2001
(73) Patentinhaber: BASF AKTIENGESELLSCHAFT, 67056 Ludwigshafen (DE)
(72) Erfinder: HALBRITTER, Klaus, D-69124 Heidelberg (DE); STERZEL, Hans-Josef, D-67125 Dannstadt-Schauernheim (DE); TRAGUT, Christian, D-67157 Wachenheim (DE); FREUDENTHALER, Eva, D-67063 Ludwigshafen (DE)
(74) Vertreter: Isenbruck, Günter, Dr.
(86) Internationale Anmeldenummer: EP0000771
(87) Internationale Veröffentlichungsnummer: WO00046218

(56) Entgegenhaltungen:
- US-A- 4 555 522
- VAN WOERDEN H F: "ORGANIC SULFITES" CHEMICAL REVIEWS,US,AMERICAN CHEMICAL SOCIETY. EASTON, Bd. 93, 1963, Seiten 557-571, XP000916509 ISSN: 0009-2665 in der Anmeldung erwähnt
- RIEMSCHNEIDER R ET AL: "BUTEN(2)-DIOL-1,4-CYCL. SULFIT" ZEITSCHRIFT FUER NATURFORSCHUNG, Bd. 15B, 1960, Seiten 552-554, XP000916507 ISSN: 0341-0382 in der Anmeldung erwähnt
- DATABASE XFIRE [Online] BEILSTEIN INSTITUT FÜR LITERATUR DER ORGANISCHEN CHEMIE Reaction ID 178517, XP002142282 & VOSS ET AL: JUSTUS LIEBIGS ANN. CHEM., Bd. 485, 1931, Seite 275
- DATABASE XFIRE [Online] BEILSTEIN INSTITUT FÜR LITERATUR DER ORGANISCHEN CHEMIE Reaction ID 1581878, XP002142283 & OLSEN R J ET AL: J.ORG.CHEM., Bd. 48, Nr. 21, 1983, Seiten 3847-3848,
- MARCH J: "ORGANIC CHEMISTRY. REACTIONS, MECHANISMS, AND STRUCTURE" 1992 , JOHN WILEY , NEW YORK XP002142281 *KAPITEL 0-23* Seite 398 -Seite 397

## Beschreibung

Die Erfindung betrifft ein Verfahren zur Herstellung von Dimethylsulfit, aus einem höheren Dialkylsulfit oder einem cyclischen Alkylensulfit und Methanol.

Ein gebräuchliches Verfahren zur Herstellung von Dialkylsulfiten, insbesondere von Dimethylsulfit, ist die Umsetzung von Thionylchlorid mit einem Alkohol, im Falle von Dimethylsulfit, mit Methanol. Die Herstellung von Dimethylsulfit durch eine solche Umsetzung ist beispielsweise in W. Voss et al., Justus Liebigs Ann. Chem. 485 (1931) 258 - 283 beschrieben.

Die Umsetzung mit Thionylchlorid hat jedoch den Nachteil, daß bei der Reaktion große Mengen Chlorwasserstoff entstehen, was hinsichtlich der Korrosion an die zu verwendenden Werkstoffe besondere Anforderungen stellt. Wird der Chlorwasserstoff nicht rasch genug aus dem Reaktionsgemisch entfernt, so reagiert dieser in einer Nebenreaktion mit dem gebildeten Dialkylsulfit zu Alkylchlorid, Alkohol und Schwefeldioxid.

Es ist weiterhin bekannt, daß Dialkylsulfite und cyclische Alkylensulfite mit Alkoholen Umesterungsreaktionen eingehen können, bei denen - den eingesetzten Alkoholen entsprechende - umgeesterte Alkylsulfite entstehen. Diese Umesterungsreaktion ist eine Gleichgewichtsreaktion. Sie wird durch Säuren und Basen katalysiert, läuft bei erhöhter Temperatur jedoch auch ohne Katalysator ab.

R. Riemschneider et al., Z. Naturforschung 15 b (1960) 552 - 554 beschreiben die Herstellung eines cyclischen Sulfits, des 2-Buten-1,4-diols, durch Umsetzung von Dimethylsulfit mit dem entsprechenden Diol. Um das Gleichgewicht der Reaktion auf die Seite des gewünschten Produktes zu verschieben, wird entstehendes Methanol fortwährend destillativ entfernt.

H. F. van Woerden, Chem. Rev. 93 (1963) 557 - 571 beschreibt die Umesterung von Dimethylsulfit mit Ethylenglykol zu Ethylensulfit und Methanol ohne Katalysator. Um Ethylensulfit in guten Ausbeuten zu erhalten, wird das entstehende Methanol kontinuierlich zur Verschiebung des sich einstellenden Gleichgewichts in Richtung des Produktes entfernt.

Ein solches Vorgehen ist nur dann möglich, wenn der sich bildende Alkohol einen niedrigeren Siedepunkt aufweist als der eingesetzte Alkohol.

Bei der Herstellung von Dimethylsulfit durch Umsetzung von cyclischen Alkylensulfiten wird Methanol als Alkohol eingesetzt. Der sich bildende Alkohol weist daher stets einen höheren Siedepunkt auf als das eingesetzte Methanol. Bei einer destillativen Gleichgewichtsverschiebung, wie sie in der Literatur beschrieben ist, würde zuerst das Methanol entfernt und so die Rückreaktion zu den Ausgangsprodukten, anstelle einer Gleichgewichtsverschiebung in Richtung des Produktes, begünstigt.

Aufgabe der vorliegenden Erfindung ist es daher, ein Verfahren zur Herstellung von Dimethylsulfit - ausgehend von cyclischen Alkylensulfiten - bereitzustellen, in dem Dimethylsulfit in guten Ausbeuten und in hoher Reinheit erhalten werden kann.

Diese Aufgabe wird durch ein Verfahren zur Herstellung von Dimethylsulfit durch Umesterung eines cyclischen Alkylensulfits mit mindestens 2 Kohlenstoffatomen mit Methanol, gegebenenfalls in Gegenwart eines Katalysators, gelöst. Das erfindungsgemäße Verfahren ist dadurch gekennzeichnet, daß das Verfahren kontinuierlich in einer Kolonne durchgeführt wird.

Das erfindungsgemäße Verfahren hat den Vorteil, daß nicht, wie bei der Umsetzung von Thionylchlorid mit Alkoholen, Chlorwasserstoff entsteht, der die verwendeten Werkstoffe angreifen und mit dem gebildeten Produkt reagieren kann. Der entstehende Alkohol kann für verschiedene Anwendungen weiterverwendet werden.

Das erfindungsgemäße Verfahren wird vorzugsweise im Gegenstromverfahren durchgeführt, wobei Methanol im unteren Teil der Kolonne zugegeben wird und im oberen Teil der Kolonne zugegebenem cyclischem Alkylensulfit entgegenströmt.

Der bei der Umesterung dem eingesetzten cyclischen Alkylensulfit entsprechende freigesetzte höhere Alkohol wird über den Sumpf der Kolonne, und das entstehende Dimethylsulfit wird, zusammen mit nicht umgesetztem Methanol, über den Kopf der Kolonne kontinuierlich abgezogen.

Das cyclische Alkylensulfit wird im Folgenden als Ausgangssulfit bezeichnet.

Bei den freigesetzten Alkoholen handelt es sich um Diole.

Die Kolonne, in der die Umesterung durchgeführt wird, kann sowohl eine Bodenkolonne als auch eine Packungskolonne sein. Vorzugsweise handelt es sich bei der Kolonne um eine Bodenkolonne, z. B. eine Glockenbodenkolonne.

Das Ausgangssulfit wird im allgemeinen im oberen Teil der Kolonne flüssig zudosiert.

Vorzugsweise wird das erfindungsgemäße Verfahren in Gegenwart eines Katalysators durchgeführt, um die Umesterung zu beschleunigen. Dieser ist besonders bevorzugt in dem eingesetzten Ausgangssulfit löslich. Dadurch kann der Katalysator dem eingesetzten Ausgangssulfit zugesetzt und zusammen mit diesem in die Kolonne eingeführt werden. Besonders bevorzugt ist als Katalysator Methansulfonsäure.

Bei einer ausreichenden Verweilzeit auf den Böden der Kolonne kann auf einen Katalysator verzichtet werden.

Zur einfachen Zudosierung wird der Katalysator im Ausgangssulfit gelöst. Das zur Umesterung eingesetzte Methanol wird im allgemeinen im unteren Teil der Kolonne dampfförmig zudosiert.

Das Ausgangssulfit und Methanol werden im allgemeinen in einem molaren Verhältnis von 1 zu mindestens 2 auf die Kolonne gegeben. Bevorzugt wird ein stöchiometrischer Überschuß von 5 - 20 : 1, besonders bevorzugt von 10 : 1 Methanol eingesetzt. Das nicht umgesetzte Methanol wird anschließend vorzugsweise in das Verfahren zurückgeführt.

Im Sumpf der Kolonne liegt im wesentlichen das nach der Umesterung aus dem entsprechenden Ausgangssulfit freigesetzte Diol vor. Daneben kann der Sumpf geringe Mengen an Kondensationsprodukten des Diols enthalten sowie gegebenenfalls eingesetzten Katalysator.

Im Kopf der Kolonne fällt das Dimethylsulfit (Reaktionsprodukt) zusammen mit nicht umgesetztem Methanol im allgemeinen gasförmig an.

Das erhaltene Dimethylsulfit wird bevorzugt in einer zweiten Kolonne von dem gleichzeitig anfallenden Methanol getrennt. Der Produktstrom, enthaltend Dimethylsulfit und Methanol, wird flüssig oder bevorzugt gasförmig auf die zweite Kolonne überführt. Die zweite Kolonne kann eine Boden- oder Packungskolonne sein.

Die zweite Kolonne wird im allgemeinen bei einem Kopfdruck von 100 mbar bis 5 bar, bevorzugt von 500 bis 1000 mbar, besonders bevorzugt bei 800 mbar betrieben.

Die Sumpftemperatur in der zweiten Kolonne beträgt bei 800 mbar 100 bis 110°C und das Rücklaufverhältnis wird so eingestellt, daß die Kopftemperatur 55 bis 60°C beträgt.

Im Kopf der zweiten Kolonne fällt Methanol an, das vorzugsweise in die erste Kolonne zurückgeführt wird. Die Rückführung des Methanols erfolgt vorzugsweise dampfförmig, um Energie zu sparen. Die erste und die zweite Kolonne sind vorzugsweise über Zuleitungen miteinander verbunden.

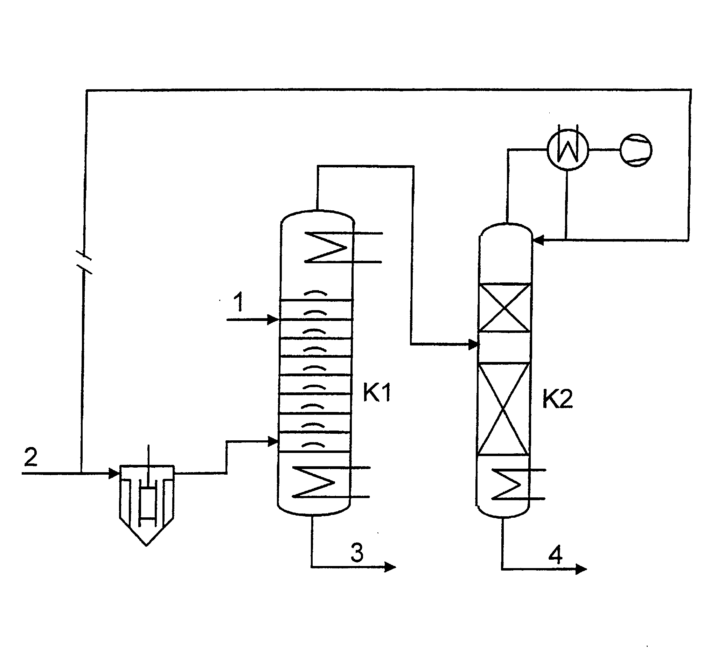

In einer besonders bevorzugten Ausführungsform wird das erfindungsgemäße Verfahren in einem Kolonnenverbund durchgeführt (siehe Fig. 1), umfassend:
- eine erste Kolonne (Kl), in der die erfindungsgemäße Umesterung durchgeführt wird und
- eine zweite Kolonne (K2), in der das am Kopf der ersten Kolonne anfallende Gemisch aus Dimethylsulfit und Methanol getrennt wird, worin
- die erste Kolonne einen Zulauf für das Ausgangssulfit und gegebenenfalls den Katalysator (1) aufweist, der in den oberen Teil der Kolonne führt; einen Zulauf für Methanol (2), der in den unteren Teil der Kolonne führt, sowie einen Ablauf für das im Sumpf anfallende Diol, Katalysator und gegebenenfalls Kondensationsprodukte des Diols (3), und
- eine Verbindung vom Kopf der ersten Kolonne in den mittleren Teil der zweiten Kolonne vorhanden ist, durch die das Gemisch aus Dimethylsulfit und Methanol der zweiten Kolonne zugeführt wird, worin
- die zweite Kolonne einen Ablauf für im Sumpf anfallendes Dimethylsulfit (Produkt) (4) aufweist, sowie
- eine Verbindung vom Kopf der zweiten Kolonne zum unteren Teil der ersten Kolonne, durch die das am Kopf der zweiten Kolonne anfallende überschüssige Methanol in die erste Kolonne zurückgeführt werden kann.

Die Dosierung von frischem Methanol in die erste Kolonne wird vorzugsweise über das Temperaturprofil im unteren Bereich der zweiten Kolonne geregelt. Steigt die Temperatur im unteren Bereich der zweiten Kolonne zu sehr an, so fehlt Methanol im Kolonnenverbund und muß entsprechend nachgeregelt werden.

Als cyclisches Alkylensulfit wird vorzugsweise ein Alkylensulfit mit 2 bis 6 Kohlenstoffatomen, besonders bevorzugt mit 2 bis 4 Kohlenstoffatomen eingesetzt. Ganz besonders bevorzugt werden Ethylensulfit oder Propylensulfit eingesetzt.

Während andere als Ausgangssulfite verwendbare cyclische Alkylensulfite im wesentlichen durch Umsetzung von Thionylchlorid und dem entsprechenden Alkohol zugänglich sind, verbunden mit den eingangs erwähnten Nachteilen, können Alkylensulfite der allgemeinen Formel I 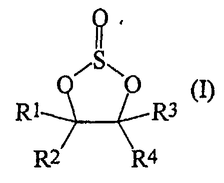 unter anderem ausgehend von den entsprechenden Epoxiden II 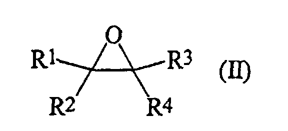 und Schwefeldioxid nach literaturbekannten Verfahren hergestellt werden. In den allgemeinen Formeln I und II sind R¹, R², R³ und R⁴ unabhängig voneinander Wasserstoff, Aryl- oder Alkylreste, bevorzugt Wasserstoff oder Methylreste. Ganz besonders bevorzugt werden Ethylensulfit und Propylensulfit eingesetzt.

Das bei der erfindungsgemäßen Umsetzung von Alkylensulfit und Methanol gebildete Diol kann nach Aufreinigung weiteren Verwendungszwecken zugeführt werden.

So findet beispielsweise das bei der Umsetzung von Ethylensulfit und Methanol gebildete Ethylenglycol in der Technik vielseitig Verwendung, beispielsweise als Komponente für Kühlflüssigkeiten, Medium zur Wärmeübertragung, hydraulische Flüssigkeit, Lösungsmittel oder als Ausgangsmaterial für weitere Synthesen.

In der beiliegenden Zeichnung ist in Figur 1 ein Verfahrensschema zur Durchführung des erfindungsgemäßen Verfahrens dargestellt. Darin bedeuten:

| | |
|---|---|
| K1 | Kolonne zur Durchführung der Umesterung |
| | |
| K2 | Kolonne zur Abtrennung von Methanol aus einem Methanol/Dimethylsulfit-Gemisch |
| | |
| 1 | Ausgangssulfit (= höheres Dialkylsulfit oder cyclisches Alkylensulfit) und Katalysator, gegebenenfalls gelöst in Methanol |
| | |
| 2 | Methanol |
| | |
| 3 | entstehender (höherer) Alkohol oder Diol |
| | |
| 4 | Dimethylsulfit |

Das nachfolgende Beispiel erläutert die Erfindung zusätzlich.

### Beispiel

Auf den 40sten Boden einer Glockenbodenkolonne (60 Böden, ∅ 43 mm) wurde ein Strom von 100 g/h Ethylensulfit und 0,44 g/h Methansulfonsäure flüssig zugefahren (Strom 1). Im Sumpf der Kolonne wurde temperaturgeregelt ein Strom von 400 g/h dampfförmigem Methanol zudosiert. Der Großteil des Methanols wurde aus der zweiten Kolonne zurückgeführt und temperaturgeregelt mit frischem Methanol (60 g/h) ergänzt (Strom 2). Der Sumpf der Kolonne wurde auf 192°C geheizt und standgeregelt abgepumpt (Strom 3). Er bestand überwiegend aus Ethylenglykol neben wenig Oligoethylenglykol und Katalysator. Die Brüden der Kolonne wurden mittels Rückflußkühler auf 75°C gekühlt und in eine zweite Kolonne (Packungskolonne, Ø 43 mm, 2 m Laborgewebepackung, 1000 cm²/cm³ Oberfläche) gefahren. Diese wurde bei 800 mbar Kopfdruck betrieben. Der Sumpf der zweiten Kolonne wurde auf 105°C aufgeheizt. Er enthielt das Dimethylsulfit und wurde standgeregelt ausgetragen (Strom 4). Das Rücklaufverhältnis wurde so eingestellt, daß die Temperatur im oberen Teil der Kolonne 58°C betrug.

## Patentansprüche

1. Verfahren zur Herstellung von Dimethylsulfit durch Umesterung eines cyclischen Alkylensulfits mit mindestens 2 Kohlenstoffatomen mit Methanol, gegebenenfalls in Gegenwart eines Katalysators, **dadurch gekennzeichnet, daß** das Verfahren kontinuierlich in einer Kolonne als Gegenstromverfahren durchgeführt wird, in dem Methanol im unteren Teil der Kolonne zugegeben wird und im oberen Teil der Kolonne zugegebenem cyclischem Alkylensulfit entgegenströmt.

2. Verfahren nach Anspruch 1, **dadurch gekennzeichnet, daß** ein in dem cyclischen Alkylensulfit löslicher Katalysator eingesetzt wird.

3. Verfahren nach Anspruch 2, **dadurch gekennzeichnet, daß** Methansulfonsäure als Katalysator eingesetzt wird.

4. Verfahren nach einem der Ansprüche 1 bis 3, **dadurch gekennzeichnet, daß** das cyclische Alkylensulfit und Methanol in einem Verhältnis von 1 zu mindestens 2 auf die Kolonne gegeben werden.

5. Verfahren nach einem der Ansprüche 1 bis 4, **dadurch gekennzeichnet, daß** das Dimethylsulfit in einer zweiten Kolonne von dem nicht umgesetzten, zusammen mit dem Dimethylsulfit über den Kopf der Kolonne abgezogenen, Methanol getrennt wird.

6. Verfahren nach Anspruch 5, **dadurch gekennzeichnet, daß** die erste und die zweite Kolonne über Zuleitungen miteinander verbunden sind.

7. Verfahren nach einem der Ansprüche 1 bis 6, **dadurch gekennzeichnet, daß** das cyclische Alkylensulfit 2 bis 6 Kohlenstoffatome aufweist.

8. Verfahren nach Anspruch 7, **dadurch gekennzeichnet, daß** Ethylensulfit oder Propylensulfit als cyclisches Alkylensulfit eingesetzt werden.

## Claims

1. A process for the preparation of dimethyl sulfite by transesterification of a cyclic alkylene sulfite of at least 2 carbon atoms with methanol, in the presence or absence of a catalyst, wherein the process is carried out continuously in a column by the countercurrent method, in which methanol is added in the lower part of the column and flows countercurrent to cyclic alkylene sulfite added in the upper part of the column.

2. A process as claimed in claim 1, wherein a catalyst soluble in the cyclic alkylene sulfite is used.

3. A process as claimed in claim 2, wherein the catalyst used is methanesulfonic acid.

4. A process as claimed in any of claims 1 to 3, wherein the cyclic alkylene sulfite and methanol are added to the column in a ratio of 1 to at least 2.

5. A process as claimed in any of claims 1 to 4, wherein the dimethyl sulfite is separated in a second column from the unconverted methanol taken off together with the dimethyl sulfite via the top of the column.

6. A process as claimed in claim 5, wherein the first and the second columns are connected to one another via feed lines.

7. A process as claimed in any of claims 1 to 6, wherein the cyclic alkylene sulfite has 2 to 6 carbon atoms.

8. A process as claimed in claim 7, wherein the cyclic alkylene sulfite used is ethylene sulfite or propylene sulfite.

## Revendications

1. Procédé pour la fabrication de diméthylsulfite par transformation de l'estérification d'un sulfite d'alkylène cyclique, comportant au moins 2 atomes de carbone, avec du méthanol, éventuellement en présence d'un catalyseur, **caractérisé en ce que** le procédé est réalisé en continu dans une colonne en tant que procédé à contre-courant, dans lequel du méthanol est ajouté à la partie inférieure de la colonne et s'écoule en sens inverse du sulfite d'alkylène cyclique ajouté à la partie supérieure de la colonne.

2. Procédé selon la revendication 1, **caractérisé en ce qu'**un catalyseur soluble dans le sulfite d'alkylène cyclique est utilisé.

3. Procédé selon la revendication 2, **caractérisé en ce que** de l'acide méthane-sulfonique est utilisé comme catalyseur.

4. Procédé selon l'une des revendications 1 à 3, **caractérisé en ce que** le sulfite d'alkylène cyclique et le méthanol sont ajoutés dans la colonne dans une proportion de 1 à au moins 2.

5. Procédé selon l'une des revendications 1 à 4, **caractérisé en ce que** le diméthylsulfite est séparé, dans une seconde colonne, du méthanol non décomposé, extrait avec le diméthylsulfite par la tête de la colonne.

6. Procédé selon la revendication 5, **caractérisé en ce que** la première et la seconde colonnes sont reliées l'une à l'autre par des conduites.

7. Procédé selon l'une des revendications 1 à 6, **caractérisé en ce que** le sulfite d'alkylène cyclique présente 2 à 6 atomes de carbone.

8. Procédé selon la revendication 7, **caractérisé en ce que** du sulfite d'éthylène ou du sulfite de propylène est utilisé comme sulfite d'alkylène cyclique.
